# FASCICULE DE BREVET EUROPEEN

(11) **EP 3 173 114 B1**
(45) Date de publication et mention de la délivrance du brevet: **29.09.2021**
(21) Numéro de dépôt: 16199926.3
(22) Date de dépôt: 22.11.2016
(51) Int. Cl.: A61M 5/24, A61M 5/28

(54) **DISPOSITIF POUR RÉALISER UNE CONNEXION HERMÉTIQUE ENTRE UNE AIGUILLE ET UN CONTENEUR**
VORRICHTUNG ZUR HERSTELLUNG EINER HERMETISCH GESCHLOSSENEN VERBINDUNG ZWISCHEN EINER NADEL UND EINEM BEHÄLTER
DEVICE FOR PRODUCING A TIGHT CONNECTION BETWEEN A NEEDLE AND A CONTAINER

(30) Priorité: 26.11.2015 FR 1561413
(43) Date de publication de la demande: 31.05.2017
(73) Titulaire: Commissariat à l'Énergie Atomique et aux Énergies Alternatives, 75015 Paris (FR)
(72) Inventeur: CHARLES, Raymond, 38430 SAINT-JEAN DE MOIRANS (FR); SARRUT-RIO, Nicolas, 38180 SEYSSINS (FR)
(74) Mandataire: INNOV-GROUP

(56) Documents cités:
- WO-A1-2011/068544
- WO-A1-2011/112136
- WO-A1-2012/059455
- WO-A1-2013/190941
- WO-A1-2014/064100
- WO-A1-2015/055592
- WO-A2-2013/079652
- US-A- 3 820 652

## Description

### Domaine technique de l'invention

La présente invention se rapporte à un dispositif pour réaliser une connexion hermétique entre une aiguille et un conteneur et à un procédé pour mettre en œuvre ladite connexion hermétique à l'aide dudit dispositif. L'invention s'applique notamment dans le domaine des cartes micro-fluidiques, cassettes micro-fluidiques ou laboratoire sur puce en général.

### Etat de la technique

La micro-fluidique désigne à la fois la science qui étudie les micro-fluides et les techniques qui permettent de les maîtriser. Aujourd'hui, l'emploi de la micro-fluidique connaît un essor important. Elle est employée pour des solutions de diagnostic ou des tests, notamment dans le domaine médical. Elle est mise en œuvre dans des cartes munies de micro-canaux dans lesquels sont injectés des liquides. Chaque carte peut comporter plusieurs entrées et plusieurs sorties. Les micro-canaux sont ensuite organisés sur la carte entre les différentes entrées et sorties. Un liquide est par exemple injecté à l'aide d'une aiguille sur une entrée de la carte.

Dans certaines applications, notamment de recherche médicale, il est bien évident que toute pollution de l'extérieur vers l'intérieur doit être évitée, c'est-à-dire que la connexion de l'aiguille sur l'entrée de la carte ne doit amener aucune impureté dans les micro-canaux de la carte.

Bien entendu, il existe des solutions pour éviter toute pollution dans les micro-canaux de la carte mais ces solutions ne sont pas toujours simples à mettre en œuvre.

La demande de brevet WO2013/188703 propose une solution pour réaliser une connexion hermétique entre une aiguille et un conteneur. Cette solution utilise un manchon agencé pour recouvrir les ouïes de l'aiguille, par lesquelles le liquide est injecté ou aspiré. Lors de la connexion, l'aiguille perce une membrane du conteneur et le manchon se replie pour libérer les ouïes. Cette solution présente cependant un inconvénient en ce qu'elle nécessite une mise en place précise du manchon pour que celui-ci recouvre les ouïes de manière étanche. De plus elle oblige à glisser le manchon jusqu'à sa position finale d'obturation des ouïes, risquant de blesser le manipulateur avec l'extrémité pointue de l'aiguille.

La demande de brevet WO2012/059455 décrit une solution de connexion hermétique entre une aiguille et un conteneur permettant le mélange entre plusieurs composés. Elle utilise notamment une architecture complexe pour assurer le mélange.

Le but de l'invention est de proposer un dispositif pour réaliser une connexion hermétique entre une aiguille et le volume interne d'un conteneur, sans intrusion de particules ou organismes extérieurs, qui soit fiable, facile à mettre en œuvre et d'un coût réduit.

### Exposé de l'invention

Ce but est atteint par un dispositif pour réaliser une connexion hermétique entre un conteneur définissant un volume interne et muni d'une ouverture et une aiguille munie d'un canal interne destiné à recevoir un liquide et d'au moins une extrémité de connexion présentant une pointe et une surface externe latérale, ce dispositif comportant :
- un septum présentant au moins une membrane destinée à fermer de manière étanche ladite ouverture du conteneur et présentant une surface d'appui,
- un capuchon présentant un flan latéral agencé pour s'appliquer contre la surface externe latérale de l'extrémité de connexion de l'aiguille et une paroi d'extrémité destinée à fermer de manière étanche ledit canal de l'aiguille à son extrémité de connexion et présentant une surface de contact agencée pour s'appliquer contre ladite surface d'appui de la membrane du septum.

Avantageusement, le septum comporte un canal interne fermé à une première extrémité par ladite membrane et ouvert à une deuxième extrémité de manière à déboucher dans le volume interne du conteneur lorsque le septum est assemblé sur ledit conteneur.

Selon une particularité du dispositif, ladite surface d'appui de la membrane du septum et ladite surface de contact du capuchon sont planes.

Selon une autre particularité, ledit canal s'étend suivant un axe perpendiculaire à la surface d'appui de ladite membrane.

Préférentiellement, l'axe du canal est centré par rapport à la surface d'appui de ladite membrane.

Selon une autre particularité du dispositif, la surface d'appui de ladite membrane est formée par un disque.

Selon une autre particularité, le septum comporte une première partie à section constante, une deuxième partie à section rétrécie et des flans incurvés concaves réalisant la jonction entre la première partie et la deuxième partie.

Selon une autre particularité, la deuxième partie présente une section constante formant une paroi latérale délimitant la deuxième extrémité du canal.

L'invention concerne également un procédé pour réaliser une connexion hermétique entre une aiguille munie d'un canal destiné à recevoir un liquide et d'au moins une extrémité de connexion, et un conteneur muni d'une ouverture, ledit procédé étant mis en œuvre à l'aide du dispositif défini ci-dessus, dans lequel le capuchon est apposé à l'extrémité de connexion de l'aiguille pour fermer son canal de manière étanche et le septum est disposé pour fermer l'ouverture du conteneur de manière étanche. Ce procédé comporte les étapes suivantes :
- application de la surface de contact du capuchon contre ladite surface d'appui de la membrane du septum,
- application d'une force de translation pour percer, dans un premier temps, ladite surface d'appui du capuchon par l'extrémité de connexion de l'aiguille puis, dans un deuxième temps, ladite membrane du septum en vue de faire déboucher l'extrémité de connexion de l'aiguille à l'intérieur du conteneur.

L'invention concerne enfin l'ensemble de connexion hermétique regroupant l'aiguille, le conteneur et le dispositif de l'invention tel que décrit ci-dessus.

### Brève description des figures

D'autres caractéristiques et avantages vont apparaître dans la description détaillée qui suit faite en regard des dessins annexés dans lesquels :
- les figures 1A et 1B représentent, vus en perspective, l'aiguille et le capuchon, respectivement avant assemblage et après assemblage,
- les figures 2A et 2B représentent le septum et le conteneur, respectivement séparés et assemblés,
- les figures 3A à 3E illustrent de manière schématique les différentes étapes du procédé pour réaliser une connexion hermétique entre l'aiguille et le conteneur.

### Description détaillée d'au moins un mode de réalisation

L'invention concerne un dispositif pour réaliser une connexion hermétique entre le volume V1 interne d'un conteneur 1 et le canal interne d'une aiguille 2, telle que par exemple une aiguille d'une seringue. L'aiguille pourra notamment être associé à un consommable dans lequel on stocke un liquide de type solvant, réactif biologique ou chimique.

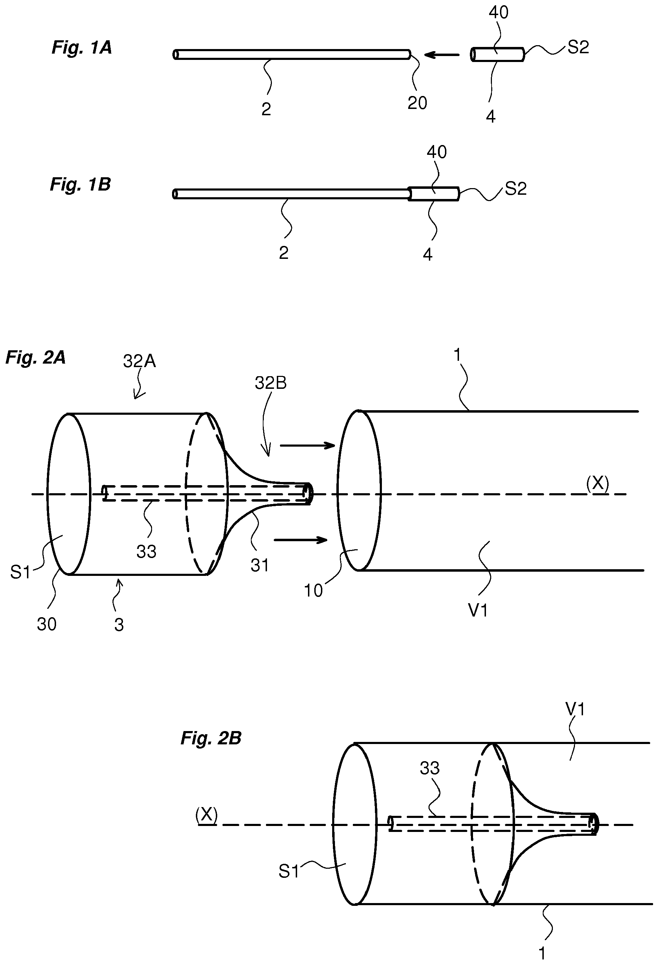

Le conteneur 1 pourra être l'entrée d'un micro-canal d'une carte micro-fluidique. En référence à la figure 2A, le conteneur 1 présente notamment une ouverture 10 pour faire communiquer son volume V1 interne avec l'extérieur.

De manière connue et en référence au figures 1A et 1B, une aiguille 2 se présente sous la forme d'une tige présentant une paroi latérale, par exemple cylindrique, définissant un canal interne débouchant vers l'extérieur par une extrémité de l'aiguille, dite extrémité de connexion. A son extrémité de connexion, l'aiguille comporte par exemple une pointe 20 agencée pour perforer une membrane. Dans le cas d'une sortie axiale, la sortie du canal est prévue à l'extrémité de la pointe et dans le cas d'une sortie radiale, la sortie du canal est réalisée sous la forme d'au moins une ouïe située en amont de la pointe.

En référence aux figures annexées, le dispositif de l'invention présente les deux parties suivantes :
- un septum 3 ayant la forme d'un bouchon présentant une forme adaptée pour fermer de manière étanche l'ouverture 10 du conteneur 1,
- un capuchon 4 destiné à être apposé à l'extrémité de connexion de l'aiguille 2 pour obturer de manière étanche la sortie du canal interne de l'aiguille 2.

De manière plus précise, en référence à la figure 2A, le septum 3 comporte au moins une membrane 30 réalisée dans un matériau suffisamment souple pour être perforé par la pointe de l'aiguille 2. Ladite membrane 30 est par exemple réalisée dans un matériau de type caoutchouc, silicone ou élastomère (EPDM). Elle comporte au moins une surface d'appui S1, préférentiellement plane, par exemple en forme de disque. Ladite surface d'appui S1 est préférentiellement centrée sur la membrane. Un indicateur visuel est par exemple représenté sur la membrane pour marquer l'emplacement de la surface d'appui.

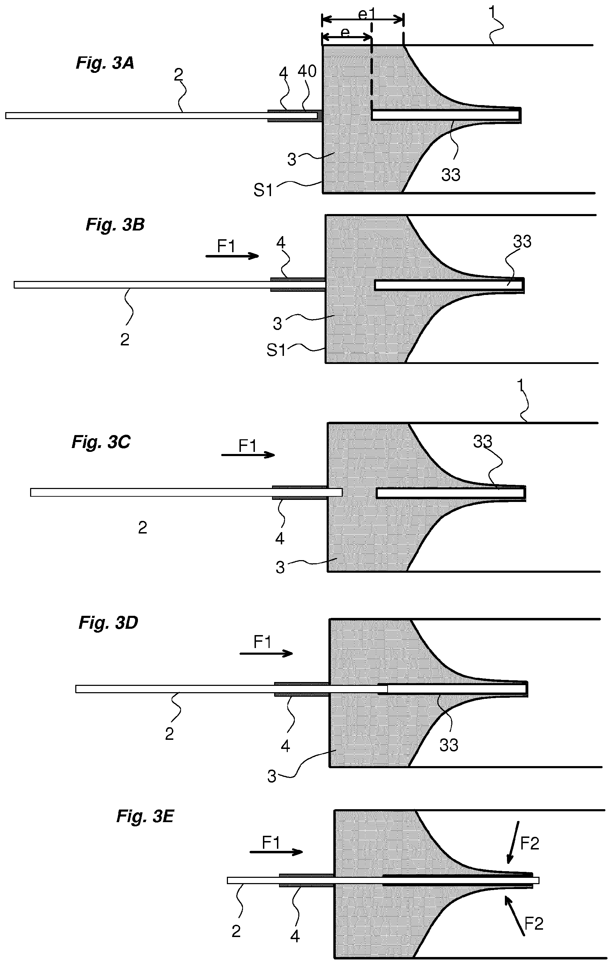

Avantageusement, le septum 3 se présente sous la forme d'une pièce monobloc. Il présente une première partie 32a de section constante incluant la membrane 30 et s'étendant sur une épaisseur e1 plus importante que l'épaisseur e de la membrane (voir figure 3A) et une deuxième partie 32b prolongeant la première partie et présentant une section rétrécie. Entre la première partie et la deuxième partie, le septum présente des flancs 31 incurvés concaves. La deuxième partie du septum 3 forme une partie proéminente en forme de tube. Le tube délimite ainsi un canal 33 interne ayant une extrémité ouverte destinée à déboucher dans le volume V1 interne du conteneur 1 et se prolongeant à travers le septum 3 suivant un axe (X) perpendiculaire à la surface d'appui S1 de la membrane 30 jusqu'à une deuxième extrémité initialement obturée par la membrane 30.

Le capuchon 4 est agencé pour recouvrir l'extrémité de connexion de l'aiguille 2 et ainsi pour obturer la sortie du canal de l'aiguille de manière étanche par rapport à l'extérieur.

En référence aux figures 1A et 1B, le capuchon 4 présente un flan latéral 40 destiné à recouvrir la surface latérale de l'aiguille 2, à son extrémité de connexion, et une paroi d'obturation appliquée contre la pointe de l'aiguille de manière à obturer son canal interne. Le flanc latéral du capuchon définit avantageusement une paroi cylindrique dont le diamètre interne au repos, avant positionnement sur l'aiguille, est avantageusement inférieur au diamètre externe de l'aiguille à son extrémité de connexion de manière à venir épouser la paroi radiale de l'aiguille à son extrémité de connexion et s'appliquer contre sa surface latérale en assurant une étanchéité lorsqu'il est positionné à l'extrémité de l'aiguille 2. Ladite paroi d'obturation du capuchon, agencée transversalement par rapport à sa paroi cylindrique, comporte une surface de contact S2 externe, préférentiellement plane, agencée pour venir en appui contre la surface d'appui S1 de la membrane 30 du septum 3 et une surface interne, opposée à sa surface externe, préférentiellement parallèle à celle-ci, et destinée à venir en appui contre la pointe de l'aiguille pour obturer le canal interne de l'aiguille. Le capuchon 4 sera par exemple réalisé en une seule pièce dans un matériau souple, par exemple de type caoutchouc, silicone ou élastomère (EPDM). La paroi d'obturation est formée de manière à présenter une épaisseur adaptée pour être perforée par l'extrémité de l'aiguille en exerçant une force de translation de l'aiguille suivant un axe sensiblement perpendiculaire à la surface de contact S2 externe qu'elle définit.

A l'aide du dispositif ainsi défini, la mise en œuvre de la connexion hermétique entre l'aiguille 2 et le conteneur 1 est réalisée de la manière suivante :
- le capuchon 4 est positionné à l'extrémité de connexion de l'aiguille 2 de manière à obturer la sortie de son canal de manière étanche, le flanc latéral du capuchon (4) venant épouser la paroi latérale externe de l'aiguille à son extrémité de connexion,
- le septum 3 est positionné pour obturer de manière étanche l'ouverture 10 du conteneur 1, la surface d'appui S1 de la membrane 30 étant orientée vers l'extérieur,
- l'aiguille 2 est avancée de sorte que la surface de contact S2 plane du capuchon 4 soit en appui contre la surface d'appui S1 plane de la membrane 30 du septum 3 (figure 3A),
- une force de translation F1 est exercée dans l'axe de l'aiguille,
- dans un premier temps, la pointe de l'aiguille perce la paroi d'obturation du capuchon (figure 3B),
- dans un deuxième temps, en poursuivant l'exercice de la force F1, la pointe de l'aiguille perce la membrane 30 du septum 3, la surface de contact S2 du capuchon est alors maintenue en appui contre la surface d'appui S1 plane de la membrane (figure 3C),
- en poursuivant encore l'exercice de la force de translation F1, la pointe de l'aiguille 2 débouche dans le canal 33 du septum, après avoir percé la membrane 30 sur son épaisseur e (figure 3D),
- en poursuivant la translation de l'aiguille, celle-ci débouche dans le volume V1 interne du conteneur 1 en débouchant du canal 33 du septum (figure 3E),
- par ses flans 31, la paroi latérale du septum, notamment sur sa deuxième partie, enserre l'aiguille 2 dans toutes les directions radiales, réalisant l'étanchéité (figure 3E).

De manière avantageuse, la pression du liquide présent dans le conteneur concourt à l'étanchéité du septum sur l'aiguille en exerçant des forces radiales (flèches F2) contre les flans concaves du septum et la deuxième partie 32b du septum. Le tube réalisé en sortie du septum 3 est choisi suffisamment fin et souple afin de pouvoir se déformer sous cette pression.

On comprend également que, lors de l'enfoncement de l'aiguille 2, les efforts de frottement du capuchon 4 contre l'aiguille 2, ont tendance à plaquer ledit capuchon 4 contre la membrane du septum.

Avantageusement, des moyens de guidage peuvent être employés pour guider l'aiguille vers la membrane et s'assurer que celle-ci vienne la percer suivant l'axe du canal 33 du septum.

La solution de l'invention présente ainsi de nombreux avantages. Elle permet de garantir une étanchéité parfaite de l'extérieur vers l'intérieur et de l'intérieur vers l'extérieur, avant, pendant et à l'issue de la connexion. Avant connexion, le canal de l'aiguille et le conteneur sont en effet fermés de manière étanche, respectivement par le capuchon et par le septum. Et après connexion, cette étanchéité est maintenue grâce au procédé mis en œuvre ci-dessus.

La solution de l'invention sera particulièrement adaptée pour être employée dans des applications de type micro-fluidique dans lesquelles il est nécessaire d'injecter un liquide à une entrée d'une carte micro-fluidique. Bien entendu, toute autre application nécessitant l'établissement d'une connexion hermétique entre une aiguille et un conteneur pourra être envisagée.

## Revendications

1. Ensemble de connexion hermétique comprenant un conteneur (1) définissant un volume (V1) interne et muni d'une ouverture (10) et une aiguille (2) munie d'un canal interne destiné à recevoir un liquide et d'au moins une extrémité de connexion présentant une pointe et une surface externe latérale, **caractérisé en ce qu'**il comporte un dispositif pour réaliser une connexion hermétique entre ledit conteneur (1) et ladite aiguille (2), ledit ensemble étant **caractérisé en ce qu'**il comporte :
- un septum (3) présentant au moins une membrane (30) destinée à fermer de manière étanche ladite ouverture (10) du conteneur et présentant une surface d'appui (S1),
- un capuchon (4) présentant un flan latéral agencé pour s'appliquer contre la surface externe latérale de l'extrémité de connexion de l'aiguille et une paroi d'extrémité perforable destinée à fermer de manière étanche ledit canal interne de l'aiguille (2) à son extrémité de connexion et présentant une surface de contact (S2) agencée pour s'appliquer contre ladite surface d'appui (S1) de la membrane (30) du septum, et **en ce que** :
- le septum (3) comporte un canal (33) interne au septum fermé à une première extrémité par ladite membrane (30) et ouvert à une deuxième extrémité de manière à déboucher dans le volume (V1) interne du conteneur (1) lorsque le septum (3) est assemblé sur ledit conteneur.

2. Ensemble selon la revendication 1, **caractérisé en ce que** ladite surface d'appui (S1) de la membrane (30) du septum et ladite surface de contact (S2) du capuchon (4) sont planes.

3. Ensemble selon la revendication 2, **caractérisé en ce que** ledit canal s'étend suivant un axe perpendiculaire à la surface d'appui (S1) de ladite membrane (30).

4. Ensemble selon la revendication 3, **caractérisé en ce que** l'axe du canal (33) est centré par rapport à la surface d'appui (S1) de ladite membrane.

5. Ensemble selon la revendication 4, **caractérisé en ce que** la surface d'appui (S1) de ladite membrane est formée par un disque.

6. Ensemble selon l'une des revendications 1 à 5, **caractérisé en ce que** le septum (3) comporte une première partie (32a) à section constante, une deuxième partie (32b) à section rétrécie et des flancs (31) incurvés concaves réalisant la jonction entre la première partie et la deuxième partie.

7. Ensemble selon la revendication 6, **caractérisé en ce que** la deuxième partie présente une section constante formant une paroi latérale délimitant la deuxième extrémité du canal.

8. Ensemble selon l'une des revendications 1 à 7, **caractérisé en ce que** la paroi d'extrémité perforable du capuchon (4) comporte une surface interne opposée à sa surface de contact et agencée pour venir en contact avec la pointe de l'aiguille (2) pour obturer le canal interne de l'aiguille (2).

9. Procédé pour réaliser une connexion hermétique entre une aiguille munie d'un canal destiné à recevoir un liquide et d'au moins une extrémité de connexion, et un conteneur muni d'une ouverture, **caractérisé en ce qu'**il est mis en oeuvre à l'aide de l'ensemble de connexion hermétique défini dans l'une des revendications 1 à 8, dans lequel le capuchon (4) est apposé à l'extrémité de connexion de l'aiguille pour fermer son canal de manière étanche et le septum (3) est disposé pour fermer l'ouverture du conteneur de manière étanche, ledit procédé étant également **caractérisé en ce qu'**il comporte les étapes suivantes :
- application de la surface de contact (S2) du capuchon contre ladite surface d'appui (S1) de la membrane du septum,
- application d'une force de translation (F1) pour percer, dans un premier temps, ladite surface d'appui du capuchon par l'extrémité de connexion de l'aiguille puis, dans un deuxième temps, ladite membrane (30) du septum en vue de faire déboucher l'extrémité de connexion de l'aiguille à l'intérieur du conteneur.

## Patentansprüche

1. Anordnung zur Erzielung einer hermetischen Verbindung, umfassend einen Behälter (1), der ein inneres Volumen (V1) definiert und mit einer Öffnung (10) versehen ist, und eine Nadel (2), die mit einem inneren Kanal, der zur Aufnahme einer Flüssigkeit bestimmt ist, und mindestens einem Verbindungsende versehen ist, das eine Spitze und eine äußere Seitenfläche aufweist, **dadurch gekennzeichnet, dass** sie eine Vorrichtung zur Herstellung einer hermetischen Verbindung zwischen dem Behälter (1) und der Nadel (2) aufweist, wobei die Anordnung **dadurch gekennzeichnet ist, dass** sie Folgendes aufweist:
ein Septum (3), das mindestens eine Membran (30) aufweist, die dazu bestimmt ist, die Öffnung (10) des Behälters dicht zu verschließen, und eine Anlagefläche (S1) aufweist,
- eine Kappe (4), die einen seitlichen Flansch, der dazu angeordnet ist, sich an die äußere Seitenfläche des Verbindungsendes der Nadel anzulegen, und eine perforierbare Endwand aufweist, die dazu bestimmt ist, den inneren Kanal der Nadel (2) an ihrem Verbindungsende dicht zu verschließen, und eine Kontaktfläche (S2) aufweist, die dazu angeordnet ist, sich an die Anlagefläche (S1) der Membran (30) des Septums anzulegen, und dass
- das Septum (3) einen Kanal (33) im Innern des Septums aufweist, der an einem ersten Ende durch die Membran (30) verschlossen ist und an einem zweiten Ende offen ist, so dass er in das innere Volumen (V1) des Behälters (1) mündet, wenn das Septum (3) an dem Behälter angebracht ist.

2. Anordnung nach Anspruch 1, **dadurch gekennzeichnet, dass** die Anlagefläche (S1) der Membran (30) des Septums und die Kontaktfläche (S2) der Kappe (4) eben sind.

3. Anordnung nach Anspruch 2, **dadurch gekennzeichnet, dass** sich der Kanal entlang einer senkrecht zu der Anlagefläche (S1) der Membran (30) verlaufenden Achse erstreckt.

4. Anordnung nach Anspruch 3, **dadurch gekennzeichnet, dass** die Achse des Kanals (33) bezüglich der Anlagefläche (S1) der Membran zentriert ist.

5. Anordnung nach Anspruch 4, **dadurch gekennzeichnet, dass** die Anlagefläche (S1) der Membran durch eine Scheibe gebildet wird.

6. Anordnung nach einem der Ansprüche 1 bis 5, **dadurch gekennzeichnet, dass** das Septum (3) einen ersten Teil (32a) mit konstantem Querschnitt, einen zweiten Teil (32b) mit verengtem Querschnitt und konkave gekrümmte Flanken (31) aufweist, die den Übergang zwischen dem ersten Teil und dem zweiten Teil bilden.

7. Anordnung nach Anspruch 6, **dadurch gekennzeichnet, dass** der zweite Teil einen konstanten Querschnitt aufweist, der eine Seitenwand bildet, die das zweite Ende des Kanals begrenzt.

8. Anordnung nach einem der Ansprüche 1 bis 7, **dadurch gekennzeichnet, dass** die perforierbare Endwand der Kappe (4) eine innere Fläche aufweist, die ihrer Kontaktfläche gegenüberliegt und dazu ausgelegt ist, mit der Spitze der Nadel (2) in Kontakt zu kommen, um den inneren Kanal der Nadel (2) zu verschließen.

9. Verfahren zur Herstellung einer hermetischen Verbindung zwischen einer Nadel, die mit einem zur Aufnahme einer Flüssigkeit bestimmten Kanal und mindestens einem Verbindungsende versehen ist, und einem Behälter, der mit einer Öffnung versehen ist, **dadurch gekennzeichnet, dass** es mit Hilfe der Anordnung zur Erzielung einer hermetischen Verbindung nach einem der Ansprüche 1 bis 8 ausgeführt wird, bei dem die Kappe (4) an dem Verbindungsende der Nadel angefügt wird, um ihren Kanal dicht zu verschließen, und das Septum (3) angeordnet wird, um die Öffnung des Behälters dicht zu verschließen, wobei das Verfahren auch **dadurch gekennzeichnet ist, dass** es die folgenden Schritte aufweist:
- Anlegen der Kontaktfläche (S2) der Kappe an die Anlagefläche (S1) der Membran des Septums,
- Ausüben einer Translationskraft (F1), um als erstes die Anlagefläche der Kappe mit dem Verbindungsende der Nadel zu durchstechen und dann als zweites die Membran (30) des Septums, so dass das Verbindungsende der Nadel im Inneren des Behälters mündet.

## Claims

1. Assembly for achieving hermetic connection, comprising a container (1) defining an internal volume (V1) and equipped with an aperture (10) and a needle (2) equipped with an internal channel, which internal channel is intended to receive a liquid, and with at least one connecting end having a tip and a lateral external surface, **characterized in that** it comprises a device for producing a hermetic connection between said container (1) and said needle (2), said assembly being **characterized in that** it comprises:
- a septum (3) comprising at least one membrane (30), which membrane is intended to close in a seal-tight manner said aperture (10) of the container and comprising a bearing surface (S1),
- a cap (4) having a lateral flange arranged to be applied against the lateral external surface of the connecting end of the needle and a perforable end wall intended to close in a seal-tight manner said internal channel of the needle (2) at its connecting end and having a contact surface (S2) arranged to be applied against said bearing surface (S1) of the membrane (30) of the septum, and **in that**:
- the septum (3) comprises a channel (33) internal to the septum that is closed at a first end by said membrane (30) and open at a second end so as to emerge into the internal volume (V1) of the container (1) when the septum (3) is assembled on said container.

2. Assembly according to Claim 1, **characterized in that** said bearing surface (S1) of the membrane (30) of the septum and said contact surface (S2) of the cap (4) are planar.

3. Assembly according to Claim 2, **characterized in that** said channel extends along an axis perpendicular to the bearing surface (S1) of said membrane (30).

4. Assembly according to Claim 3, **characterized in that** the axis of the channel (33) is centred with respect to the bearing surface (S1) of said membrane.

5. Assembly according to Claim 4, **characterized in that** the bearing surface (S1) of said membrane is formed by a disc.

6. Assembly according to one of Claims 1 to 5, **characterized in that** the septum (3) comprises a first portion (32a) of constant cross section, a second portion (32b) of narrow cross section and concave curved sidewalls (31) forming the join between the first portion and second portion.

7. Assembly according to Claim 6, **characterized in that** the second portion has a constant cross section forming a lateral partition bounding the second end of the channel.

8. Assembly according to one of Claims 1 to 7, **characterized in that** the perforable end wall of the cap (4) comprises an internal surface opposite its contact surface and arranged to make contact with the tip of the needle (2) to obturate the internal channel of the needle (2).

9. Method for producing a hermetic connection between a needle equipped with a channel intended to receive a liquid and with at least one connecting end, and a container equipped with an aperture, **characterized in that** it is implemented using the assembly for achieving hermetic connection defined in one of Claims 1 to 8, wherein the cap (4) is placed on the connecting end of the needle to close its channel in a seal-tight manner and the septum (3) is placed to close the aperture of the container in a seal-tight manner, said method also being **characterized in that** it comprises the following steps:
- applying the contact surface (S2) of the cap against said bearing surface (S1) of the membrane of the septum,
- applying a translational force (F1) to pierce, initially, said bearing surface of the cap with the connecting end of the needle then, subsequently, said membrane (30) of the septum, with a view to making the connecting end of the needle emerge into the interior of the container.
